# EUROPEAN PATENT APPLICATION

(11) **EP 4 588 455 A1**
(43) Date of publication of application: **23.07.2025**
(21) Application number: 23864733.3
(22) Date of filing: 13.09.2023
(51) Int. Cl.: A61C 7/08

(54) **DENTAL INSTRUMENT AND PREPARATION METHOD THEREFOR**

(30) Priority: 13.09.2022 CN 202211112137
(71) Applicant: Shanghai Ea Medical Instruments Company Limited, Shanghai 200433 (CN)
(72) Inventor: LI, Junsheng, Shanghai 200433 (CN); ZHOU, Ketuo, Shanghai 200433 (CN)
(74) Representative: Winter, Brandl - Partnerschaft mbB
(86) International application number: PCT/CN2023/118626
(87) International publication number: WO 2024/056001

(57) **Abstract**

A dental appliance and preparation method thereof. The dental appliance comprises a shell-type dental aligner, a functional attachment, and an adhesive layer for bonding the shell-type dental aligner with the functional attachment. The bond strength between the adhesive layer and the shell-type dental aligner and the functional attachment is between 5MPa~20MPa, the ratio R1 between the flexural modulus of the adhesive layer and the flexural modulus of the shell-type dental aligner is between 0.05~0.15, and/or the ratio R2 between the flexural stiffness K1 of the bonding portion of the functional attachment and the flexural stiffness K2 of the bonding portion of the shell-type dental aligner is between 0.05~0.15. The present application adjusts the rigidity of the adhesive layer and/or the local rigidity of the bonding portion of the functional attachment, so that the mechanical energy generated by the bending deformation of the shell-type dental aligner can be effectively absorbed through the elastic deformation of the adhesive layer and the bonding portion of the functional attachment. These bending stresses will not be transmitted to the bonding surface in large proportions, thereby preventing damage to the bonding surface and improving bonding reliability.

## Description

### TECHNICAL FIELD

The present application is in the field of dental appliance technology and relates to a dental appliance and preparation method thereof.

### BACKGROUND

With the development of dental orthodontics, shell-type dental aligners have become increasingly popular due to their advantages of convenient wearing, aesthetics, and ease of daily oral care. To enhance the correction effect, many functional attachments are usually combined with shell-type dental aligners to achieve functions such as traction, forward guidance of the mandible, and bite opening.

Currently, these functional attachments are typically combined with oral appliances through bonding. Considering the usage scenarios of these functional attachments, improving the clinical reliability of bonding is very necessary.

### SUMMARY

To solve one of the problems existing in the prior art, an object of the present application is to provide a dental appliance and preparation method thereof.

To achieve one of the above objects, the present application adopts the following technical solution.

A dental appliance , comprising a shell-type dental aligner , a functional attachment and an adhesive layer for bonding the shell-type dental aligner with the functional attachment, characterized in that the bond strength between the adhesive layer and the shell-type dental aligner and between the adhesive layer and the functional attachment are both greater than 5MPa, a ratio R1 between a flexural modulus of the adhesive layer and a flexural modulus of the shell-type dental aligner is between 0.05~0.15, and/or a ratio R2 between a flexural stiffness K1 of a bonding portion of the functional attachment and a flexural stiffness K2 of a bonding portion of the shell-type dental aligner is between 0.05~0.15.

Furthermore, the R1 is between 0.05~0.15, and an elastic modulus of the functional attachment is between 300MPa~2000MPa.

Furthermore, a thickness of the adhesive layer is between 0.05mm~0.2mm.

Furthermore, the flexural modulus of the shell-type dental aligner is between 1000MPa~2000MPa; or, the flexural modulus of the adhesive layer is between 100MPa~500MPa.

Furthermore, when R2 is between 0.05~0.15, R1>0.15.

Furthermore, the attachment bonding portion comprises at least one reduced material portion.

Furthermore, a reduction ratio of the reduced material portion is not less than 25%.

Furthermore, the reduced material portion is located in a middle region of the bonding portion of the functional attachment.

Furthermore, the reduced material portion is located in an edge region of the bonding portion of the functional attachment.

Furthermore, the functional attachment includes an attachment bonding portion for bonding with the shell-type dental aligner and an auxiliary correction portion for auxiliary correction, wherein at least one reduced material portion is arranged circumferentially along a connection position where the attachment bonding portion connects with the auxiliary correction portion.

Furthermore, the functional attachment includes an attachment bonding portion for bonding with the shell-type dental aligner and an auxiliary correction portion for auxiliary correction, wherein at least two reduced material portions are evenly arranged circumferentially along a connection position where the attachment bonding portion connects with the auxiliary correction portion.

Furthermore, the functional attachment includes an attachment bonding portion for bonding with the shell-type dental aligner and an auxiliary correction portion for auxiliary correction, wherein at least one reduced material portion is evenly distributed on the attachment bonding portion.

Furthermore, the reduced material portion is a thinned portion, and a thickness of the thinned portion is less than a thickness of other regions on the bonding portion of the functional attachment.

Furthermore, the thinned portion is recessed from at least one of a bonding side and a non-bonding side of the attachment bonding portion.

Furthermore, the reduced material portion is a hollow portion, and the hollow portion penetrates through the bonding portion of the functional attachment along at least one direction.

Furthermore, the hollow portion penetrates through the bonding portion of the functional attachment along a direction parallel to a bonding surface, or the hollow portion penetrates through the bonding portion of the functional attachment along a direction perpendicular to the bonding surface.

Furthermore, an elastic modulus of the bonding portion of the functional attachment is less than an elastic modulus of a non-bonding portion.

Furthermore, the elastic modulus of the bonding portion of the functional attachment is between 50MPa~150MPa.

Furthermore, a flexural modulus of the non-bonding portion of the functional attachment is between 300MPa~2000MPa.

Furthermore, the bond strength between the adhesive layer and the shell-type dental aligner, and between the adhesive layer and the functional attachment are both between 5MPa~20MPa.

Furthermore, the functional attachment comprises at least one of a traction button, a bracket, a bite plate, a twin-block, a guide plane, or a reinforcing rib.

A method for preparing a dental appliance, characterized in that it comprises the following steps:
when bond strengths between an adhesive layer and a shell-type dental aligner, and between the adhesive layer and a functional attachment are both greater than 5MPa, obtaining flexural moduli of the shell-type dental aligner and the adhesive layer;
if a ratio R1 between the flexural modulus of the adhesive layer and the flexural modulus of the shell-type dental aligner is between 0.05~0.15, bonding the functional attachment; if R1>0.15, adjusting rigidity of a bonding portion of the functional attachment to make a ratio R2 between a flexural stiffness K1 of the bonding portion of the functional attachment and a flexural stiffness K2 of a bonding portion of the shell-type dental aligner between 0.05~0.15, then bonding the functional attachment.

A method for preparing a dental appliance, characterized in that it comprises the following steps:
when bond strengths between an adhesive layer and a shell-type dental aligner, and between the adhesive layer and a functional attachment are both greater than 5MPa, obtaining flexural stiffnesses of the bonding portion of the shell-type dental aligner and the bonding portion of the functional attachment;
if a ratio R2 between a flexural stiffness K1 of the bonding portion of the functional attachment and a flexural stiffness K2 of the bonding portion of the shell-type dental aligner is between 0.05~0.15, bonding the functional attachment to the bonding portion of the shell-type dental aligner using an adhesive; if R2>0.15, adjusting rigidity of the adhesive layer for bonding the functional attachment to make a ratio R1 between a flexural modulus of the adhesive layer and a flexural modulus of the shell-type dental aligner between 0.05~0.15.

A method for preparing a dental appliance, characterized in that it comprises the following steps:
forming an adhesive layer on a bonding portion of a shell-type dental aligner;
bonding a functional attachment on the adhesive layer;
wherein bond strengths between the adhesive layer and the shell-type dental aligner, and between the adhesive layer and the functional attachment are both greater than 5MPa, a ratio R1 between a flexural modulus of the adhesive layer and a flexural modulus of the shell-type dental aligner is between 0.05~0.15, and/or a ratio R2 between a flexural stiffness K1 of a bonding portion of the functional attachment and a flexural stiffness K2 of the bonding portion of the shell-type dental aligner is between 0.05~0.15.

Furthermore, a thickness of the adhesive layer is between 0.05mm~0.2mm.

Furthermore, at least one reduced material portion is formed on the attachment bonding portion.

Furthermore,t the reduced material portion is a thinned portion, and a thickness of the thinned portion is less than a thickness of other regions on the bonding portion of the functional attachment; or, the reduced material portion is a hollow portion, and the hollow portion penetrates through the bonding portion of the functional attachment along at least one direction.

Furthermore, an elastic modulus of the bonding portion of the functional attachment is less than an elastic modulus of a non-bonding portion.

Furthermore, the bond strength between the adhesive layer and the shell-type dental aligner, and between the adhesive layer and the functional attachment are both between 5MPa~20MPa.

Furthermore, the functional attachment comprises at least one of a traction button, a bracket, a bite plate, a twin-block, a guide plane, or a reinforcing rib.

Compared with the prior art, under the condition of certain bonding strength between the adhesive and the shell-type dental aligner and functional attachment, the present application adjusts the rigidity of the adhesive layer and/or the local rigidity of the bonding portion of the functional attachment, so that the mechanical energy generated by the bending deformation of the shell-type dental aligner can be effectively absorbed through the elastic deformation of the adhesive layer and/or the bonding portion of the functional attachment. These bending stresses will not be transmitted to the bonding surface in large proportions, thereby effectively preventing damage to the bonding surface and improving bonding reliability.

### BRIEF DESCRIPTION OF THE DRAWINGS

FIG. 1 is a structural diagram of a traditional traction button.
FIG. 2 is a structural diagram of FIG.1 from another angle.
FIG. 3 is a structural diagram of a traction button according to one embodiment of the present application.
FIG. 4 is a structural diagram of a traction button with hollow portions according to another embodiment of the present application.
FIG. 5 is a structural diagram of FIG. 4 from another angle.
FIG. 6 is a structural diagram of FIG. 4 from another angle.
FIG. 7 is a structural diagram of a traction button with thinned portions according to another embodiment of the present application.
FIG. 8 is a structural diagram of FIG. 7 from another angle.
FIG. 9 is a fitting diagram of a traditional bite plate with a shell-type dental aligner.
FIG. 10 is a structural diagram of a bite plate according to one embodiment of the present application.
FIG. 11 is a structural diagram of a bite plate according to another embodiment of the present application.
FIG. 12 is a fitting diagram of the bite plate of FIG. 11 with a shell-type dental aligner.

### DETAILED DESCRIPTION OF EMBODIMENTS

The following detailed description of the present application will be made with reference to the specific embodiments shown in the drawings. However, these embodiments do not limit the present application and any structural, methodological, or functional variations made by those skilled in the art based on these embodiments are included within the scope of protection of the present application.

The inventors found that shell-type dental aligners often undergo certain bending deformation during clinical use, including bending deformation of bonding platforms, functional attachments and bonding surfaces, which are the main reasons for bonding failure of functional attachments. Based on this finding, the present application improves clinical bonding reliability by adjusting the rigidity at the bonding location.

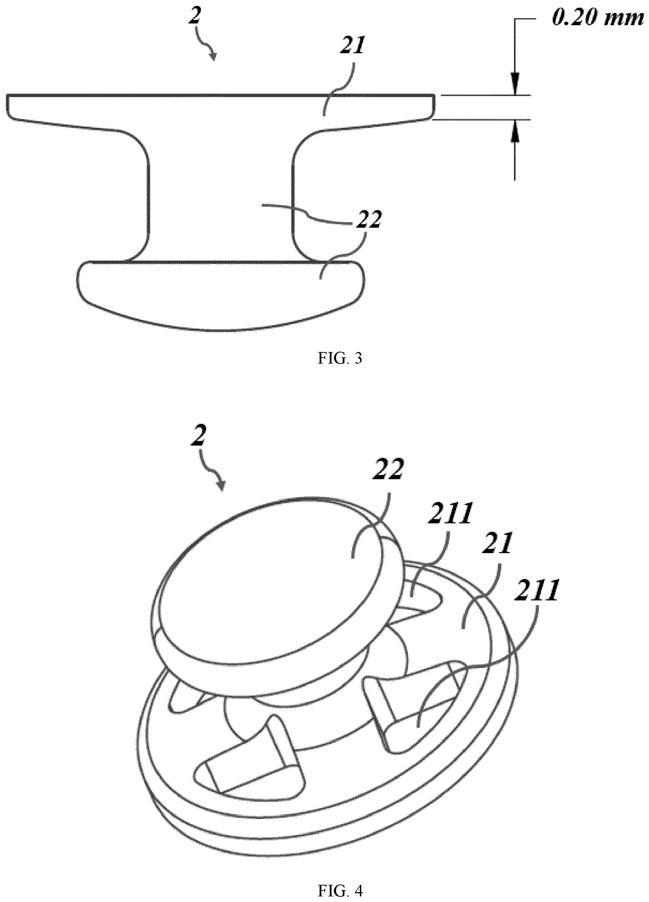

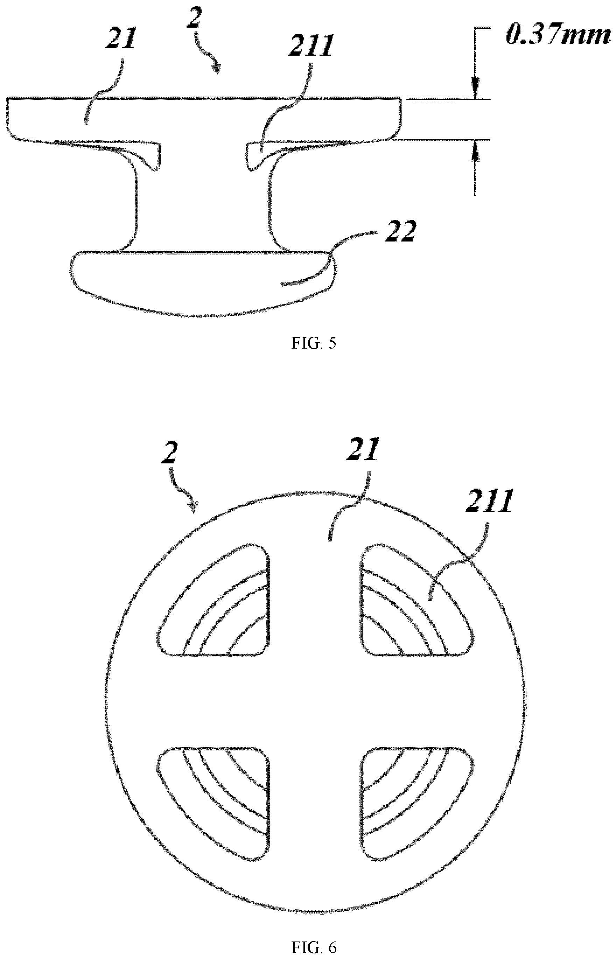

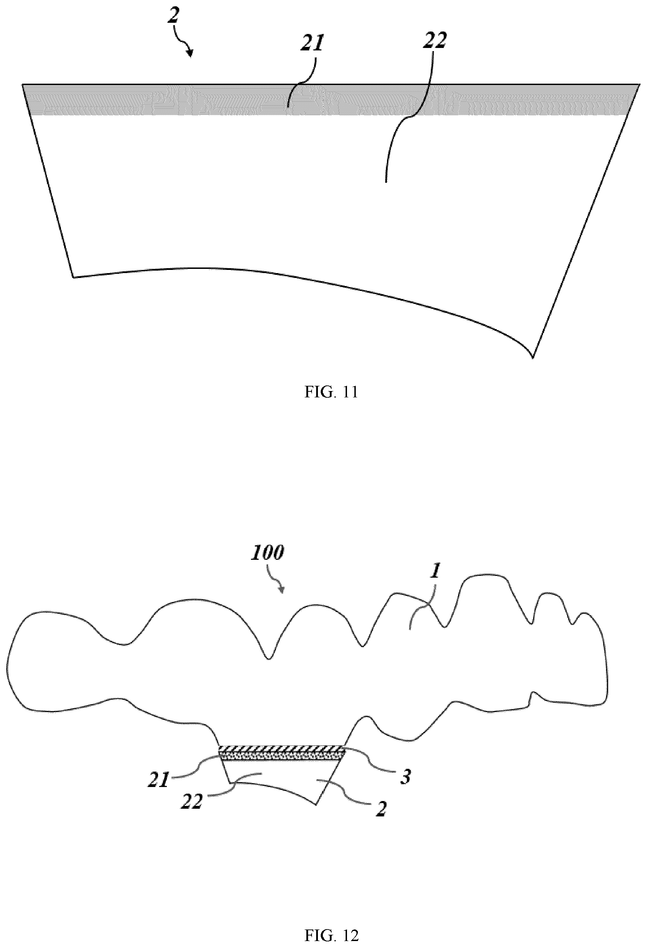

Please refer to Figures 3-8 and 10-12, which show a preferred embodiment of the dental appliance 100 of the present application. The dental appliance 100 includes a shell-type dental aligner 1, a functional attachment 2 and an adhesive layer 3. Where the adhesive layer 3 bonds the shell-type dental aligner 1 with the functional attachment 2.

The shell-type dental aligner 1 is used to be worn on one or more teeth. The shell-type dental aligner 1 includes an inner surface facing the teeth and an outer surface away from the teeth, where the inner surface forms a receiving cavity for accommodating all or part of the teeth.

In a specific embodiment, the shell-type dental aligner 1 is an invisible aligner. The shell-type dental aligner 1 is made of plastic material and is transparent and colorless. Specifically, the shell-type dental aligner 1 can be prepared using any material and any method known in the art.

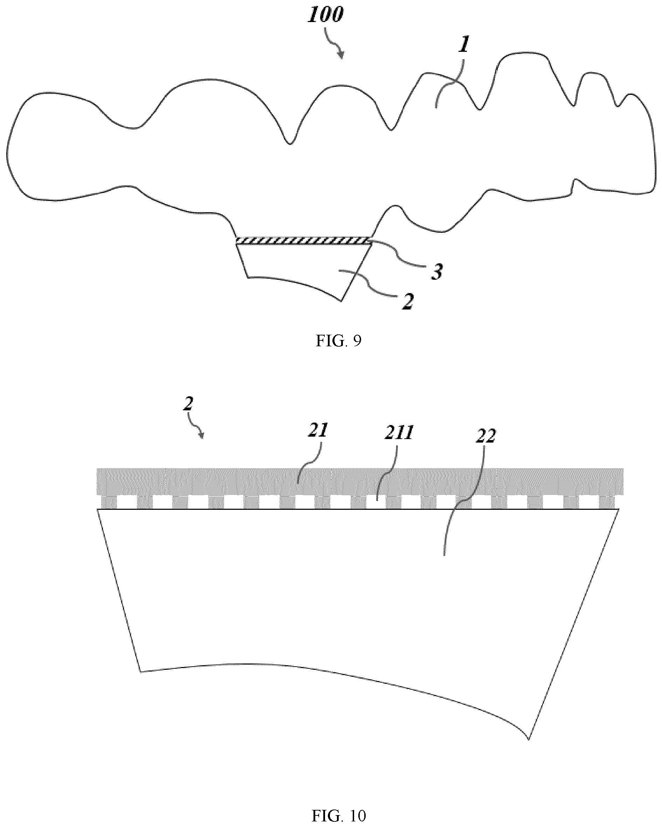

The functional attachment 2 includes but is not limited to a traction button, a bracket, a bite plate, a twin-block, a guide plane or a reinforcing rib. The functional attachment 2 can enhance the correction effect of the shell-type dental aligner 1, or the functional attachment 2 can add correction functions to the shell-type dental aligner 1. The present application mainly uses the traction button shown in Figures 3-8 and the bite plate shown in Figures 10-12 as examples for explanation, and other functional attachments will not be elaborated one by one.

Specifically, the functional attachment 2 includes an attachment bonding portion 21 for bonding with the shell-type dental aligner 1 and an auxiliary correction portion 22 that provides auxiliary correction function. Those skilled in the art can understand that the attachment bonding portion 21 is the bonding portion of the functional attachment 2, which is the local area for bonding with the adhesive. Other structures except for the attachment bonding portion 21 are collectively referred to as the auxiliary correction portion 22, and the detailed substructures of the auxiliary correction portion 22 will not be described in detail.

For some functional attachments 2, the attachment bonding portion 21 and the auxiliary correction portion 22 are integral with no obvious boundary line between them, such as the bite plate shown in Figures 9 and 11-12. For such functional attachments 2, the present application defines the attachment bonding portion 21 as the part near the bonding surface within a predetermined thickness. In one embodiment, the part with a predetermined thickness between 0.15mm~2mm is defined as the attachment bonding portion 21.

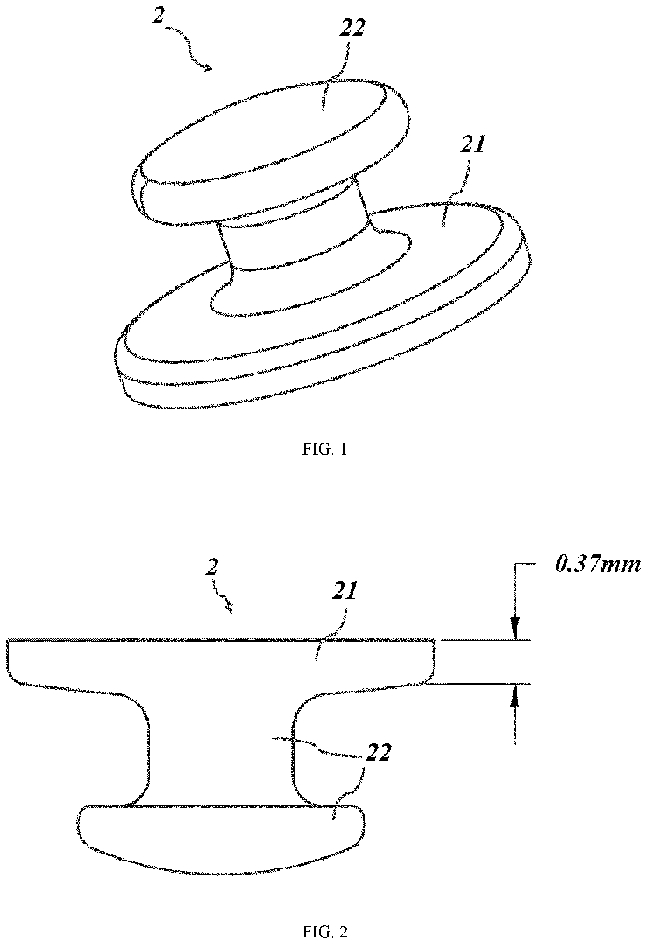

For some functional attachments 2, the attachment bonding portion 21 and the auxiliary correction portion 22 are two separate structures, such as the traction button shown in Figures 1-8.

The adhesive layer 3 is formed by curing adhesive of a certain thickness. The adhesive layer 3 is preferably food grade, medical grade, or biocompatible adhesive, environmentally friendly, and suitable for appliances placed in the oral cavity.

The inventors further found that the bond strength between the adhesive layer 3 and the shell-type dental aligner 1 and functional attachment 2 is one of the main factors determining bonding reliability. This bond strength includes the bond strength between the adhesive and the shell-type dental aligner 1, and between the adhesive and the functional attachment 2.

When the bond strength between the adhesive layer 3 and the shell-type dental aligner 1 and functional attachment 2 is less than 5MPa, the bond strength is too small to meet reliability requirements. Therefore, the present application selects adhesive that forms an adhesive layer 3 with bond strength greater than 5MPa with both the shell-type dental aligner 1 and functional attachment 2.

Preferably, the adhesive layer 3 has bond strength between 5MPa~20MPa with both the shell-type dental aligner 1 and functional attachment 2, particularly between 5MPa~8MPa, which covers commonly used adhesives with medium bond strength. During clinical use, it may be damaged; the present application further improves bonding reliability by adjusting the local rigidity of at least one of the adhesive layer 3 and the attachment bonding portion 21 to enhance the cleavage strength or peel strength of the bond.

When the bond strength between the adhesive layer 3 and the shell-type dental aligner 1 and functional attachment 2 is greater than 20MPa, the adhesive's bond strength is already strong enough, but it is preferred to adopt the solution of the present application to further improve reliability.

In the first type of embodiment, when the bond strength between the adhesive layer 3 and the shell-type dental aligner 1, and between the adhesive layer 3 and the functional attachment 2 is between 5MPa~20MPa, bonding reliability can be improved by adjusting the rigidity of the adhesive layer 3.

Specifically, the present application appropriately reduces the rigidity of the adhesive layer 3, so that the mechanical energy generated by the bending deformation of the shell-type dental aligner 1 can be effectively absorbed through the elastic deformation of the adhesive layer 3. These bending stresses will not be transmitted to the bonding surface in large proportions, thereby effectively preventing damage to the bonding surface and improving bonding reliability.

Further research found that: the lower the ratio R1 between the flexural modulus of the adhesive layer 3 and the flexural modulus of the shell-type dental aligner 1, the higher the clinical reliability.

When the ratio R1 between the flexural modulus of the adhesive layer 3 and the flexural modulus of the shell-type dental aligner 1 is lower than 0.05, the material of the adhesive layer 3 is too soft, affecting the transmission of correction force, and the shell-type dental aligner 1 cannot achieve effective correction.

When R1 is between 0.05~0.15, the rigidity of the adhesive layer 3 is moderate. On one hand, it provides effective bond strength between the shell-type dental aligner 1 and the functional attachment 2. On the other hand, as an absorption buffer layer for the deformation mechanical energy of the shell-type dental aligner 1 and the functional attachment 2, it can achieve relatively high clinical reliability of bonding performance without special adjustment to the local rigidity of the attachment bonding portion 21; that is, the elastic modulus of the functional attachment 2 only needs to be adjusted according to the requirements of correction function, without special adjustment for bonding reliability, for example, the elastic modulus of the functional attachment 2 can be between 300MPa~2000MPa.

Preferably, R1 is between 0.06~0.12, particularly between 0.08~0.10. The rigidity of the adhesive layer 3 is moderate, which can both effectively transmit correction force and buffer-absorb the deformation mechanical energy of the shell-type dental aligner 1 and functional attachment 2, resulting in high bonding reliability.

Specifically, the flexural modulus of the shell-type dental aligner 1 is between 1000MPa~2000MPa. Or, the flexural modulus of the adhesive layer 3 is between 100MPa~500MPa.

The inventors further found that the ratio R1 between the flexural modulus of the adhesive layer 3 and the flexural modulus of the shell-type dental aligner 1 can be controlled through the selection of adhesive and thickness control of the adhesive layer 3. Given the limited types of adhesives and the difficulty and long cycle of adhesive development, the present application preferably controls the thickness of the adhesive layer 3.

In the present application, the thickness of the adhesive layer 3 is between 0.05mm~0.2mm, preferably 0.05mm~0.1mm. When the adhesive layer 3 is too thin, its own strength is insufficient, resulting in weak bond strength. When the adhesive layer 3 is too thick, it will not qualitatively change the bonding reliability but will waste material.

When R1>0.15, the reliability of adhesive layer 3 bonding is relatively reduced. At this time, the local rigidity of the attachment bonding portion 21 needs to be reduced to a certain range to ensure bonding reliability.

Specifically, the adjustment of local rigidity of the attachment bonding portion 21 can refer to the following second type of embodiment.

In the second type of embodiment, when the bond strength between the adhesive layer 3 and the shell-type dental aligner 1, and between the adhesive layer 3 and the functional attachment 2 are both between 5MPa~20MPa, the local rigidity of the attachment bonding portion 21 can also be adjusted to improve bonding reliability.

The smaller the ratio R2 between the flexural stiffness K1 of the attachment bonding portion 21 and the flexural stiffness K2 of the bonding portion of the shell-type dental aligner 1, the higher the clinical reliability. Where flexural stiffness K=EI, E=flexural modulus, I=moment of inertia of cross-section=bh³/12, b=cross-section width, h=cross-section height; for bonding portions with irregular shapes, their flexural stiffness can be calculated through integration.

When R2<0.05, the material of the functional attachment 2 is too soft, affecting the transmission of correction force, and the shell-type dental aligner 1 cannot achieve effective correction effect.

Preferably, R2 is between 0.05~0.15, where the rigidity of the attachment bonding portion 21 is moderate. On one hand, the attachment bonding portion 21 performs bonding, on the other hand, the attachment bonding portion 21 serves as an absorption buffer layer for the deformation mechanical energy of the shell-type dental aligner 1 and functional attachment 2, achieving relatively high clinical reliability of functional attachment 2 bonding. On this basis, the flexural modulus of the adhesive layer 3 can be disregarded, meaning reliable bonding can be achieved whether R1 is between 0.05~0.15 or R1>0.15.

The present application appropriately reduces the local rigidity of the attachment bonding portion 21, so that the mechanical energy generated by the bending deformation of the shell-type dental aligner 1 can be effectively absorbed through the elastic deformation of the attachment bonding portion 21. These bending stresses will not be transmitted to the bonding surface in large proportions, thereby effectively preventing damage to the bonding surface and improving bonding reliability.

Methods to reduce the local rigidity of the attachment bonding portion 21 include but are not limited to: structural improvements and material improvements.

First, structural improvements include but are not limited to: reducing the thickness or material of the attachment bonding portion 21 to reduce the flexural resistance of the attachment bonding portion 21 structure.

In one embodiment, comparing Figures 1-3, the thickness of the entire attachment bonding portion 21 of the functional attachment 2 is reduced to lower the flexural stiffness of the attachment bonding portion 21. This improvement method is preferably suitable for functional attachments 2 where the attachment bonding portion 21 has a clear boundary line with the auxiliary correction portion 22.

Referring to Figure 3, the thickness of the attachment bonding portion 21 is not greater than 0.2mm, preferably between 0.15mm~0.2mm. Compared to the traditional thickness of 0.37mm, the thickness of the attachment bonding portion 21 in the present application is greatly reduced, resulting in small flexural stiffness, which can well absorb the mechanical energy when the shell-type dental aligner 1 and functional attachment 2 deform.

In another embodiment, material is removed from partial areas of the attachment bonding portion 21 to reduce the flexural stiffness of the attachment bonding portion 21. That is, compared to the original complete attachment bonding portion 21, the attachment bonding portion 21 includes at least one reduced material portion.

The material reduction ratio, which is the proportion of material reduced by forming the reduced material portion relative to the mass of the original complete attachment bonding portion 21, determines the rigidity of the attachment bonding portion 21. The larger the reduction ratio, the lower the rigidity. When the reduction ratio is not less than 25%, bonding reliability can be effectively improved. For example, when the reduction ratio is 30%, the bonding failure rate can be reduced to 0.

Specifically, the reduced material portion can be a hollow portion 211 set on the attachment bonding portion 21. The hollow portion 211 penetrates through the attachment bonding portion 21 along at least one direction, reducing material usage and enhancing the buffering effect of attachment bonding portion 21 deformation, further improving bonding reliability.

Taking the traction button shown in Figures 4-6 as an example, the hollow portion 211 can penetrate through the attachment bonding portion 21 along the direction perpendicular to the bonding surface, i.e., the bonding direction.

Or, taking the bite plate shown in Figure 10 as an example, the hollow portion 211 can also penetrate through the attachment bonding portion 21 along the direction parallel to the bonding surface. The hollow portion 211 is located on the side of the attachment bonding portion 21 facing the auxiliary correction portion 22, equivalent to the attachment bonding portion 21 connecting with the auxiliary correction portion 22 through several spaced connecting columns. This hollow structure can undergo large deformation without breaking when the bite plate is under stress, and blocks the transmission of mechanical deformation, helping improve bonding reliability.

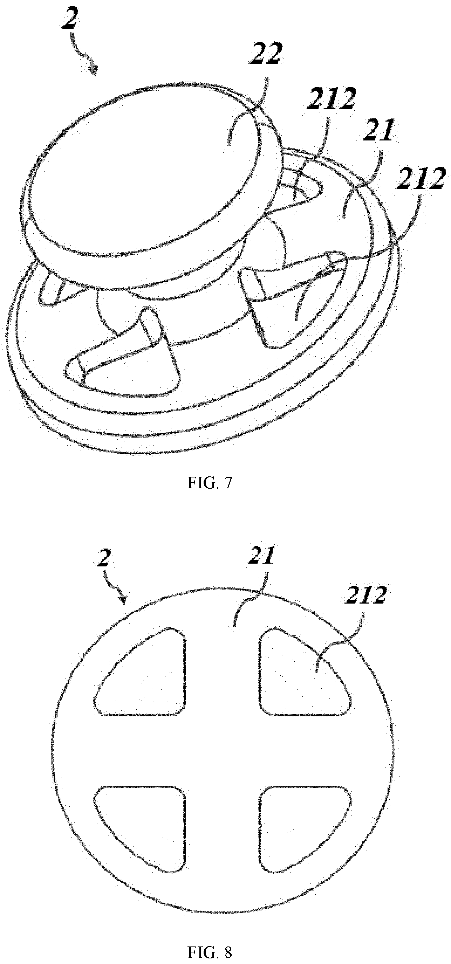

Additionally, as shown in Figures 7 and 8, the thickness of local areas on the attachment bonding portion 21 can be reduced to form the reduced material portion. That is, the reduced material portion is a thinned portion 212, whose thickness is less than that of other regions of the attachment bonding portion 21, making the entire attachment bonding portion 21 less rigid and able to deform within a certain range.

The thinned portion 212 is formed by recessing from at least one of the bonding side and non-bonding side of the attachment bonding portion 21 toward the opposite side, allowing for either unidirectional or bidirectional recessing and thinning. Those skilled in the art can understand that: the thinned portion 212 on the bonding side is recessed toward the non-bonding layer side, while the thinned portion 212 on the non-bonding side is recessed toward the bonding side.

Referring to and comparing Figures 4 and 7, the positioning of the thinned portion 212 is similar to that of the hollow portion 211. Taking the shown hollow portion 211 and thinned portion 212 as examples, the positioning of the reduced material portion is described.

The reduced material portion can be located in the middle region of the attachment bonding portion 21, where it is surrounded by other regions, and the outer periphery of the attachment bonding portion 21 remains a continuous complete structure, maintaining its inherent shape for convenient bonding. Of course, the reduced material portion can also be located in the edge region of the attachment bonding portion 21 for easy processing and forming.

Or, at least one reduced material portion is arranged circumferentially along the connection position where the attachment bonding portion 21 connects with the auxiliary correction portion 22.

Furthermore, when there are at least two reduced material portions, they are evenly arranged circumferentially along the connection position where the attachment bonding portion 21 connects with the auxiliary correction portion 22; regardless of the direction of correction force, the entire attachment bonding portion 21 maintains reliable bonding effect.

Or, at least one reduced material portion is evenly distributed on the attachment bonding portion 21, also ensuring good buffering effect when the attachment bonding portion 21 is under force in various directions, meeting clinical bonding reliability requirements.

Secondly, improvements from the material perspective. The elastic modulus of the attachment bonding portion 21 is less than that of the non-bonding portion. Different materials for the bonding and non-bonding portions can be combined through multi-component injection molding, ultrasonic welding, laser welding, induction welding, etc.

In one embodiment, the elastic modulus of the attachment bonding portion 21 is 50MPa~150MPa, while the flexural modulus of the non-bonding portion is between 300MPa~2000MPa, for example, 500MPa or 1000MPa.

Additionally, the present application can adjust the rigidity of the adhesive layer 3 as in the first type of embodiment while adjusting the local rigidity of the attachment bonding portion 21 as in the second type of embodiment, doubly improving bonding reliability. That is: any design scheme for the adhesive layer 3 in the first type of embodiment can be combined with the second type of embodiment to improve bonding reliability, and any design scheme for the attachment bonding portion 21 in the second type of embodiment can be combined with the first type of embodiment to improve bonding reliability.

The present application also provides a method for preparing a dental appliance, comprising the following steps:

Providing a shell-type dental aligner 1, adhesive, and functional attachment 2; when the bond strength between the adhesive layer 3 and the shell-type dental aligner 1, and between the adhesive layer 3 and the functional attachment 2 are both between 5MPa~20MPa, obtaining the flexural moduli of the shell-type dental aligner 1 and adhesive layer 3;

If the ratio R1 between the flexural modulus of the adhesive layer 3 and the flexural modulus of the shell-type dental aligner 1 is between 0.05~0.15, any functional attachment 2 can be directly bonded; if R1>0.15, the rigidity of the attachment bonding portion 21 needs to be adjusted to make the ratio R2 between the flexural stiffness K1 of the attachment bonding portion 21 and the flexural stiffness K2 of the bonding portion of the shell-type dental aligner 1 between 0.05~0.15, before bonding the functional attachment 2.

The present application also provides another method for preparing a dental appliance, comprising the following steps:

Providing a shell-type dental aligner 1, adhesive, and functional attachment 2; when the bond strength between the adhesive layer 3 and the shell-type dental aligner 1, and between the adhesive layer 3 and the functional attachment 2 are both between 5MPa~20MPa, obtaining the flexural stiffnesses of the bonding portion of the shell-type dental aligner 1 and the attachment bonding portion 21;

If the ratio R2 between the flexural stiffness K1 of the attachment bonding portion 21 and the flexural stiffness K2 of the bonding portion of the shell-type dental aligner 1 is between 0.05~0.15, any adhesive can be used to bond the functional attachment 2 to the bonding portion of the shell-type dental aligner 1; if R2>0.15, the rigidity of the adhesive layer 3 for bonding the functional attachment 2 needs to be adjusted to make the ratio R1 between the flexural modulus of the adhesive layer 3 and the flexural modulus of the shell-type dental aligner 1 between 0.05~0.15.

The present application also provides another method for preparing a dental appliance, comprising the following steps:
Forming an adhesive layer 3 on the bonding portion of the shell-type dental aligner 1;
Bonding a functional attachment 2 on the adhesive layer 3;
Where the bond strength between the adhesive layer 3 and the shell-type dental aligner 1, and between the adhesive layer 3 and the functional attachment 2 are both between 5MPa~20MPa, the ratio R1 between the flexural modulus of the adhesive layer 3 and the flexural modulus of the shell-type dental aligner 1 is between 0.05~0.15; and/or the ratio R2 between the flexural stiffness K1 of the attachment bonding portion 21 and the flexural stiffness K2 of the bonding portion of the shell-type dental aligner 1 is between 0.05~0.15.

Based on any of the above methods for preparing the dental appliance, the adjustment of the adhesive layer 3 can adopt any solution from the first type of embodiment above, for example: the thickness of the adhesive layer 3 is between 0.05mm~0.2mm; other details will not be elaborated.

Based on any of the above methods for preparing the dental appliance, the rigidity adjustment of the attachment bonding portion 21 can adopt any solution from the second type of embodiment above. For example: the thickness of the attachment bonding portion 21 is between 0.15mm~0.2mm. Or, the attachment bonding portion 21 includes at least one thinned portion 212, whose thickness is less than that of other regions on the attachment bonding portion 21. Or, the attachment bonding portion 21 includes at least one hollow portion 211, which penetrates through the attachment bonding portion 21 along at least one direction. Or, the elastic modulus of the attachment bonding portion 21 is less than that of the non-bonding portion; other details will not be elaborated.

Similarly, the functional attachment 2 as mentioned above includes at least one of a traction button, bracket, bite plate, twin-block, guide plane, or reinforcing rib.

The following specific examples will provide detailed explanation of the dental appliance 100 and its preparation method of the present application.

First embodiment: Taking the functional attachment 2 as a traction button as an example, under the premise that the bond strength between the adhesive and the shell-type dental aligner 1 and functional attachment 2 is 5MPa~8MPa, the bonding reliability was explored by adjusting the flexural modulus of the shell-type dental aligner 1, adhesive layer 3, and traction button, with test results shown in Table 1.

Referring to Table 1, from examples 1-7, it can be seen that when the ratio R1 between the flexural modulus of the adhesive layer 3 and the flexural modulus of the shell-type dental aligner 1 is between 0.05~0.15, the bonding reliability is not affected by the flexural modulus of the traction button. At this time, when the flexural modulus of the traction button varies between 300MPa~2000MPa, the proportion of traction button torsional failure is 0 in all cases.

From examples 8-10, it can be seen that when the ratio R1 between the flexural modulus of the adhesive layer 3 and the flexural modulus of the shell-type dental aligner 1 is >0.15, only when the rigidity of the traction button is reduced to a certain degree, for example, when the flexural modulus of the traction button is reduced from 2000MPa to 300MPa or below, making the ratio R2 between the flexural stiffness K1 of the attachment bonding portion 21 and the flexural stiffness K2 of the bonding portion of the shell-type dental aligner 1 between 0.05~0.15, does the proportion of traction button torsional failure become 0.

**Table 1**

| Experimental Methods | Evaluation of the bonding reliability of the traction button by the proportion of the traction button torsional failure | | | |
|---|---|---|---|---|
| Items | Experimental parameters | | | Test Results |
| | flexural modulus of the shell-type dental aligner (MPa) | flexural modulus of the adhesive layer (MPa) | flexural modulus of the traction button (MPa) | proportion of traction button torsional failure |
| Example 1 | 2000 | 100 | 1000 | 0% |
| Example 2 | 2000 | 100 | 2000 | 0% |
| Example 3 | 2000 | 200 | 300 | 0% |
| Example 4 | 2000 | 200 | 1000 | 0% |
| Example 5 | 2000 | 200 | 2000 | 0% |
| Example 6 | 2000 | 300 | 1000 | 0% |
| Example 7 | 2000 | 300 | 2000 | 0% |
| Example 8 | 2000 | 500 | 2000 | 30% |
| Example 9 | 2000 | 500 | 1000 | 15% |
| Example 10 | 2000 | 500 | 300 | 0% |

Second embodiment: Taking the functional attachment 2 as a traction button as an example, under the premise that the bond strength between the adhesive and the shell-type dental aligner 1 and functional attachment 2 is 5MPa~8MPa, the bonding reliability was explored by reducing the flexural stiffness of the traction button's bonding base through two methods: reducing thickness and material, with test results shown in Table 2.

Specifically, referring to Figures 1-3: when bonding the traction button to the shell-type dental aligner 1, the flexural modulus of the shell-type dental aligner 1 is 1000MPa, the flexural modulus of the adhesive layer 3 is 400MPa, R1 is 0.4; we reduced the flexural stiffness of the traction button's bonding base by reducing its thickness, with specific experimental parameters and test results shown in Table 2.

Referring to Table 2, as shown in examples 1-3, when the thickness of the traction button's bonding base is in the range of 0.25mm~0.37mm, the ratio R2 between the flexural stiffness K1 of the traction button base and the flexural stiffness K2 of the shell-type dental aligner 1's bonding portion structure is >0.15, the proportion of traction button torsional failure is greater than 10%, showing poor reliability. As shown in examples 4 and 5, when the thickness of the traction button's bonding base is in the range of 0. 15mm~0.2mm, the ratio R2 between the flexural stiffness K1 of the traction button base and the flexural stiffness K2 of the shell-type dental aligner 1's bonding portion structure is 0.05~0.12, the proportion of traction button torsional failure is 0, showing high bonding reliability. Here, the cross-section widths of the traction button base and the shell-type dental aligner 1's bonding portion are equal, the cross-section height of the traction button base is its thickness, and the cross-section height of the shell-type dental aligner 1's bonding portion structure is 0.5mm.

**Table 2**

| Experimental Methods | Evaluation of the bonding reliability of the traction button by the proportion of the traction button torsional failure | | | | | |
|---|---|---|---|---|---|---|
| Items | Experimental parameters | | | | | Test Results |
| | flexural modulus of the shell-type dental aligner (MPa) | flexural modulus of the adhesive layer (MPa) | flexural modulus of the traction button (MPa) | thickness of traction button base | the ratio between the flexural stiffness K1 of the traction button base and the flexural stiffness K2 of the shell-type dental aligner 1's bonding portion structure | proportion of traction button torsional failure |
| Example 1 | 1000 | 400 | 2000 | 0.37 | 0.8 | 20% |
| Example 2 | 1000 | 400 | 2000 | 0.3 | 0.42 | 15% |
| Example 3 | 1000 | 400 | 2000 | 0.25 | 0.25 | 10% |
| Example 4 | 1000 | 400 | 2000 | 0.2 | 0.12 | 0% |
| Example 5 | 1000 | 400 | 2000 | 0.15 | 0.05 | 0% |

The examples in Table 2 demonstrate that bonding reliability can be improved by adjusting the thickness of the attachment bonding portion 21, and when R1>0.15, R2 between 0.05~0.15 can ensure clinical bonding reliability, while R2>0.15 results in poor clinical reliability.

Third embodiment: Taking the functional attachment 2 as a traction button as an example, under the premise that the bond strength between the adhesive and the shell-type dental aligner 1 and functional attachment 2 is 5MPa~8MPa, the bonding reliability was explored by reducing the material of the traction button's bonding base, with test results shown in Table 3.

Referring to and comparing Figures 1-2, 4-8, and 10: when bonding the traction button to the shell-type dental aligner 1, the flexural modulus of the shell-type dental aligner 1 is 1000MPa, the flexural modulus of the adhesive layer 3 is 400MPa (R1 is 0.4), the flexural modulus of the traction button is 2000MPa, we can also improve bonding reliability by reducing the flexural stiffness of the traction button's bonding base through material reduction.

Specific experimental and test results are shown in Table 3. Under fixed flexural moduli of the shell-type dental aligner 1 and traction button, as the material reduction ratio of the traction button base increases, the ratio R2 between the flexural stiffness K1 of the traction button base and the flexural stiffness K2 of the shell-type dental aligner 1's bonding portion structure gradually decreases. When the reduction ratio reaches 30% or above, the proportion of traction button torsional failure reduces to 0. The material reduction ratio in this application refers to the mass ratio of reduced material to the original bonding portion material.

**Table 3**

| Experimental Methods | Evaluation of the bonding reliability of the traction button by the proportion of the traction button torsional failure | | | | | |
|---|---|---|---|---|---|---|
| Items | Experimental parameters | | | | | Test Results |
| | flexural modulus of the shell-type dental aligner (MPa) | flexural modulus of the adhesive layer (MPa) | flexural modulus of the traction button (MPa) | the material reduction ratio of the traction button base | the ratio between the flexural stiffness K1 of the traction button base and the flexural stiffness K2 of the shell-type dental aligner 1's bonding portion structure | proportion of traction button torsional failure |
| Example 1 | 1000 | 400 | 2000 | 0% | 0.8 | 20% |
| Example 2 | 1000 | 400 | 2000 | 10% | 0.5 | 17% |
| Example 3 | 1000 | 400 | 2000 | 20% | 0.25 | 10% |
| Example 4 | 1000 | 400 | 2000 | 30% | 0.13 | 0% |

Fourth embodiment: Taking the functional attachment 2 as a bite plate as an example, under the premise that the bond strength between the adhesive and the shell-type dental aligner 1 and functional attachment 2 is 5MPa~8MPa, the bonding reliability was explored by adjusting the material of the bite plate's bonding portion, with test results shown in Table 3.

Specifically, referring to Figures 9, 11-12, when bonding the bite plate to the shell-type dental aligner 1, the flexural modulus of the shell-type dental aligner 1 is 1000MPa, the flexural modulus of the adhesive is 400MPa, R1 is 0.4, which is greater than 0.15; we improved the bonding effect by using material with lower flexural modulus for the bite plate's bonding portion, with specific experimental parameters and test results shown in Table 3.

Referring to Table 4, with the flexural modulus of the bite plate's non-bonding portion at 500MPa, the flexural modulus of the bonding portion material ranges from 50MPa to 500MPa. As shown in examples 1-2, when the ratio R2 between the flexural stiffness K1 of the bite plate's bonding portion and the flexural stiffness K2 of the shell-type dental aligner 1's bonding portion structure is >0.15, the proportion of bite plate torsional failure is high, indicating poor bonding reliability. As shown in examples 3-5, when R2 is between 0.05~0.15, the proportion of bite plate torsional failure is 0, indicating high bonding reliability.

The examples in Table 4 further demonstrate that bonding reliability can be improved by adjusting the elastic modulus of the attachment bonding portion 21, and when R1>0.15, R2 between 0.05~0.15 can ensure clinical bonding reliability, while R2>0.15 results in poor clinical reliability.

**Table 4**

| Experimental Methods | Evaluation of the bonding reliability of the bite plate by the proportion of the bite plate failure | | | | |
|---|---|---|---|---|---|
| Items | Experimental parameters | | | | Test Results |
| | flexural modulus of the shell-type dental aligner (MPa) | flexural modulus of the adhesive layer (MPa) | flexural modulus of the bite plate's bonding portion (MPa) | the ratio between the flexural stiffness K1 of the bite plate's bonding portion and the flexural stiffness K2 | proportion of bite plate torsional failure |
| Example 1 | 1000 | 400 | 500 | 0.5 | 30% |
| Example 2 | 1000 | 400 | 300 | 0.3 | 15% |
| Example 3 | 1000 | 400 | 150 | 0.15 | 0% |
| Example 4 | 1000 | 400 | 100 | 0.1 | 0% |
| Example 5 | 1000 | 400 | 50 | 0.05 | 0% |

In conclusion, under the condition of fixed bond strength between the adhesive and the shell-type dental aligner 1 and functional attachment 2, the present application appropriately reduces the rigidity of the adhesive or the local rigidity of the attachment bonding portion 21, so that the mechanical energy generated by the bending deformation of the shell-type dental aligner 1 can be effectively absorbed through the elastic deformation of the adhesive layer 3 and attachment bonding portion 21. These bending stresses will not be transmitted to the bonding surface in large proportions, thereby effectively preventing damage to the bonding surface and improving bonding reliability.

It should be understood that although this specification describes the application according to embodiments, not every embodiment contains only one independent technical solution. This manner of description in the specification is merely for clarity. Those skilled in the art should consider the specification as a whole, and the technical solutions in various embodiments can also be appropriately combined to form other embodiments that can be understood by those skilled in the art.

The series of detailed descriptions listed above are merely specific explanations of feasible embodiments of this application, and they are not used to limit the scope of protection of this application. Any equivalent implementations or modifications made without departing from the technical spirit of this application should be included within the scope of protection of this application.

## Claims

1. A dental appliance , comprising a shell-type dental aligner , a functional attachment and an adhesive layer for bonding the shell-type dental aligner with the functional attachment, **characterized in that** the bond strength between the adhesive layer and the shell-type dental aligner and between the adhesive layer and the functional attachment are both greater than 5MPa, a ratio R1 between a flexural modulus of the adhesive layer and a flexural modulus of the shell-type dental aligner is between 0.05~0.15, and/or a ratio R2 between a flexural stiffness K1 of a bonding portion of the functional attachment and a flexural stiffness K2 of a bonding portion of the shell-type dental aligner is between 0.05~0.15.

2. The dental appliance according to claim 1, **characterized in that** R1 is between 0.05~0.15, and an elastic modulus of the functional attachment is between 300MPa~2000MPa.

3. The dental appliance according to claim 1, **characterized in that** a thickness of the adhesive layer is between 0.05mm~0.2mm.

4. The dental appliance according to any one of claims 1-3, **characterized in that** the flexural modulus of the shell-type dental aligner is between 1000MPa~2000MPa; or, the flexural modulus of the adhesive layer is between 100MPa~500MPa.

5. The dental appliance according to claim 1, **characterized in that** when R2 is between 0.05~0.15, R1>0.15.

6. The dental appliance according to claim 1, **characterized in that** the attachment bonding portion comprises at least one reduced material portion.

7. The dental appliance according to claim 6, **characterized in that** a reduction ratio of the reduced material portion is not less than 25%.

8. The dental appliance according to claim 6, **characterized in that** the reduced material portion is located in a middle region of the bonding portion of the functional attachment.

9. The dental appliance according to claim 6, **characterized in that** the reduced material portion is located in an edge region of the bonding portion of the functional attachment.

10. The dental appliance according to claim 6, **characterized in that** the functional attachment includes an attachment bonding portion for bonding with the shell-type dental aligner and an auxiliary correction portion for auxiliary correction, wherein at least one reduced material portion is arranged circumferentially along a connection position where the attachment bonding portion connects with the auxiliary correction portion.

11. The dental appliance according to claim 6, **characterized in that** the functional attachment includes an attachment bonding portion for bonding with the shell-type dental aligner and an auxiliary correction portion for auxiliary correction, wherein at least two reduced material portions are evenly arranged circumferentially along a connection position where the attachment bonding portion connects with the auxiliary correction portion.

12. The dental appliance according to claim 6, **characterized in that** the functional attachment includes an attachment bonding portion for bonding with the shell-type dental aligner and an auxiliary correction portion for auxiliary correction, wherein at least one reduced material portion is evenly distributed on the attachment bonding portion.

13. The dental appliance according to any one of claims 6-12, **characterized in that** the reduced material portion is a thinned portion, and a thickness of the thinned portion is less than a thickness of other regions on the bonding portion of the functional attachment.

14. The dental appliance according to claim 13, **characterized in that** the thinned portion is recessed from at least one of a bonding side and a non-bonding side of the attachment bonding portion.

15. The dental appliance according to any one of claims 6-12, **characterized in that** the reduced material portion is a hollow portion, and the hollow portion penetrates through the bonding portion of the functional attachment along at least one direction.

16. The dental appliance according to claim 15, **characterized in that** the hollow portion penetrates through the bonding portion of the functional attachment along a direction parallel to a bonding surface, or the hollow portion penetrates through the bonding portion of the functional attachment along a direction perpendicular to the bonding surface.

17. The dental appliance according to claim 1, **characterized in that** an elastic modulus of the bonding portion of the functional attachment is less than an elastic modulus of a non-bonding portion.

18. The dental appliance according to claim 17, **characterized in that** the elastic modulus of the bonding portion of the functional attachment is between 50MPa~150MPa.

19. The dental appliance according to claim 17 or 18, **characterized in that** a flexural modulus of the non-bonding portion of the functional attachment is between 300MPa~2000MPa.

20. The dental appliance according to claim 1, **characterized in that** the bond strength between the adhesive layer and the shell-type dental aligner, and between the adhesive layer and the functional attachment are both between 5MPa~20MPa.

21. The dental appliance according to claim 1, **characterized in that** the functional attachment comprises at least one of a traction button, a bracket, a bite plate, a twin-block, a guide plane, or a reinforcing rib.

22. A method for preparing a dental appliance, **characterized in that** it comprises the following steps:
when bond strengths between an adhesive layer and a shell-type dental aligner, and between the adhesive layer and a functional attachment are both greater than 5MPa, obtaining flexural moduli of the shell-type dental aligner and the adhesive layer;
if a ratio R1 between the flexural modulus of the adhesive layer and the flexural modulus of the shell-type dental aligner is between 0.05~0.15, bonding the functional attachment; if R1>0.15, adjusting rigidity of a bonding portion of the functional attachment to make a ratio R2 between a flexural stiffness K1 of the bonding portion of the functional attachment and a flexural stiffness K2 of a bonding portion of the shell-type dental aligner between 0.05~0.15, then bonding the functional attachment.

23. A method for preparing a dental appliance, **characterized in that** it comprises the following steps:
when bond strengths between an adhesive layer and a shell-type dental aligner, and between the adhesive layer and a functional attachment are both greater than 5MPa, obtaining flexural stiffnesses of the bonding portion of the shell-type dental aligner and the bonding portion of the functional attachment;
if a ratio R2 between a flexural stiffness K1 of the bonding portion of the functional attachment and a flexural stiffness K2 of the bonding portion of the shell-type dental aligner is between 0.05~0.15, bonding the functional attachment to the bonding portion of the shell-type dental aligner using an adhesive; if R2>0.15, adjusting rigidity of the adhesive layer for bonding the functional attachment to make a ratio R1 between a flexural modulus of the adhesive layer and a flexural modulus of the shell-type dental aligner between 0.05~0.15.

24. A method for preparing a dental appliance, **characterized in that** it comprises the following steps:
forming an adhesive layer on a bonding portion of a shell-type dental aligner;
bonding a functional attachment on the adhesive layer;
wherein bond strengths between the adhesive layer and the shell-type dental aligner, and between the adhesive layer and the functional attachment are both greater than 5MPa, a ratio R1 between a flexural modulus of the adhesive layer and a flexural modulus of the shell-type dental aligner is between 0.05~0.15, and/or a ratio R2 between a flexural stiffness K1 of a bonding portion of the functional attachment and a flexural stiffness K2 of the bonding portion of the shell-type dental aligner is between 0.05~0.15.

25. The method for preparing the dental appliance according to any one of claims 22-24, **characterized in that** a thickness of the adhesive layer is between 0.05mm~0.2mm.

26. The method for preparing the dental appliance according to any one of claims 22-24, **characterized in that** at least one reduced material portion is formed on the attachment bonding portion.

27. The method for preparing the dental appliance according to claim 26, **characterized in that** the reduced material portion is a thinned portion, and a thickness of the thinned portion is less than a thickness of other regions on the bonding portion of the functional attachment; or, the reduced material portion is a hollow portion, and the hollow portion penetrates through the bonding portion of the functional attachment along at least one direction.

28. The method for preparing the dental appliance according to any one of claims 22-24, **characterized in that** an elastic modulus of the bonding portion of the functional attachment is less than an elastic modulus of a non-bonding portion.

29. The method for preparing the dental appliance according to any one of claims 22-24, **characterized in that** the bond strength between the adhesive layer and the shell-type dental aligner, and between the adhesive layer and the functional attachment are both between 5MPa~20MPa.

30. The method for preparing the dental appliance according to any one of claims 22-24, **characterized in that** the functional attachment comprises at least one of a traction button, a bracket, a bite plate, a twin-block, a guide plane, or a reinforcing rib.
